(19) Europäisches Patentamt
European Patent Office
Office européen des brevets

(11) Veröffentlichungsnummer: **0 301 457 B1**

(12) **EUROPÄISCHE PATENTSCHRIFT**

(45) Veröffentlichungstag der Patentschrift: **29.09.93**

(21) Anmeldenummer: **88111956.4**

(22) Anmeldetag: **25.07.88**

(51) Int. Cl.⁵: **C07C 209/44**, C07C 227/00, C07C 229/00, C07D 217/02, C07D 209/44, C07C 211/00, C07C 211/17, C07C 211/03, C07C 229/06

# (54) Verfahren zur Herstellung von optisch aktiven sekundären Aminen.

(30) Priorität: **28.07.87 CH 2870/87**

(43) Veröffentlichungstag der Anmeldung:
**01.02.89 Patentblatt 89/05**

(45) Bekanntmachung des Hinweises auf die Patenterteilung:
**29.09.93 Patentblatt 93/39**

(84) Benannte Vertragsstaaten:
**BE CH DE FR GB IT LI NL**

(56) Entgegenhaltungen:
**EP-A- 0 104 375**
**EP-A- 0 256 982**
**DE-A- 2 456 937**
**FR-A- 2 188 597**

**JOURNAL OF MOLECULAR CATALYSIS, Band 22, 1983, Lausanne; S. VASTAG et al: "Rhodium phosphine complexes as homogeneous catalysts. 14. Assymmetric hydrogenation of a Schiff base of acetophenone - effect of phosphine and catalyst structure on enantioselectivity", Seiten 283-287**

(73) Patentinhaber: **CIBA-GEIGY AG**
**Klybeckstrasse 141**
**CH-4002 Basel(CH)**

(72) Erfinder: **Pugin, Benoit, Dr.**
**Wasserhausweg 16**
**CH-4142 Münchenstein(CH)**
Erfinder: **Ramos, Gerardo, Dr.**
**General Guisan-Strasse 35**
**CH-4144 Arlesheim(CH)**
Erfinder: **Spindler, Felix, Dr.**
**Dullikerstrasse 392**
**CH-4656 Starrkirch-Wil(CH)**

(74) Vertreter: **Zumstein, Fritz, Dr. et al**
**Bräuhausstrasse 4**
**D-80331 München (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein Verfahren zur Herstellung von optisch aktiven sekundären Aminen durch asymmetrische Hydrierung von prochiralen N-aliphatischen Ketiminen mit chiralen Iridiumdiphosphin- oder -diphosphinitkomplexen.

In der EP-A-0 104 375 sind chirale Diphosphinliganden beschrieben, deren Komplexe mit Metallen der Gruppe VIII des Periodensystems als Katalysatoren bei der asymmetrischen Hydrierung von $\alpha$-(Acylamino)-acrylsäuren verwendet werden können.

L. Markó et al. beschreiben in J. Mol. Catal. 22, S. 283 - 287 (1984) und J. Organomet. Chem. 279, S. 23 - 29 (1985) die asymmetrische Hydrierung von N-Benzyl-phenyl-methylketimin mit Rhodiumkomplexen chiraler Diphosphine bzw. Diphosphinite. Die chemische Aktivität der Komplexe ist gering und zudem sind die erzielbaren optischen Ausbeuten nicht immer reproduzierbar.

Es wurde gefunden, dass Iridiumverbindungen mit chiralen Diphosphin- oder Diphosphinitliganden geeignete homogene asymmetrische Katalysatoren für die Hydrierung von prochiralen N-aliphatischen Ketiminen sind. Diese Reaktion führt unter geringerem Druck bei hohen chemischen Umsätzen und guten optischen Ausbeuten zu optisch aktiven sekundären Aminen, die überraschend trotz ihrer starken Basizität und Nucleophilie nicht als Katalysatorgift wirken. Optisch aktiv bedeutet einen Ueberschuss eines Enantiomeren mit R-oder S-Konfiguration.

Gegenstand vorliegender Erfindung ist ein Verfahren zur Herstellung von optisch aktiven sekundären N-aliphatischen Aminen der Formel I

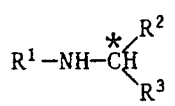

(I),

worin $R^1$ lineares oder verzweigtes $C_1$-$C_{12}$-Alkyl, Cycloalkyl mit 3 bis 8 Ring-C-Atomen, über ein C-Atom gebundenes Heterocycloalkyl mit 3 bis 8 Ringatomen und 1 oder 2 Heteroatomen aus der Gruppe O, S und $NR^4$, ein über ein Alkyl-C gebundenes $C_7$-$C_{16}$-Aralkyl oder mit dem genannten Cycloalkyl oder Heterocycloalkyl oder Heteroaryl substituiertes $C_1$-$C_{12}$-Alkyl bedeutet, die unsubstituiert oder mit $C_1$-$C_{12}$-Alkyl, $C_1$-$C_{12}$-Alkoxy, $C_1$-$C_{12}$-Alkylthio, $C_1$-$C_6$-Haloalkyl, Halogen, -OH, -CN, $C_6$-$C_{12}$-Aryl oder -Aryloxy oder -Arylthio, $C_7$-$C_{16}$-Aralkyl oder -Aralkoxy oder -Aralkylthio, wobei die Arylreste ihrerseits durch $C_1$-$C_4$-Alkyl, -Alkoxy, -Alkylthio, Halogen, -OH, -CN, $-CONR^4R^5$ oder $-COOR^4$ substituiert sein können, Sekundäramino mit 2 bis 24 C-Atomen,

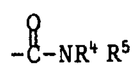

oder $-COOR^4$, wobei $R^5$ für H steht oder unabhängig die Bedeutung von $R^4$ hat und $R^4$ $C_1$-$C_{12}$-Alkyl, Phenyl oder Benzyl oder $R^4$ und $R^5$ zusammen Tetra- oder Pentamethylen oder 3-Oxapentylen sind, substituiert sein können;

$R^2$ und $R^3$ voneinander verschieden sind und gegebenenfalls mit -OH, -CN, Halogen, $C_1$-$C_{12}$-Alkoxy, Phenoxy, Benzyloxy, Sekundäramino mit 2 bis 24 C-Atomen,

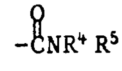

oder $-COOR^4$ substituiertes $C_1$-$C_{12}$-Alkyl oder Cycloalkyl mit 3-8 Ring-C-Atomen, gegebenenfalls wie zuvor für $R^1$ in der Bedeutung von Aralkyl und Aryl substituiertes $C_6$-$C_{12}$-Aryl oder $C_7$-$C_{16}$-Aralkyl oder $-CONR^4R^5$ oder $-COOR^4$ darstellen, worin $R^4$ und $R^5$ die zuvor angegebene Bedeutung haben; oder

$R^1$ die zuvor angegebene Bedeutung hat und $R^2$ und $R^3$ zusammen gegebenenfalls durch 1 oder 2 -O-, -S- oder $-NR^4$- unterbrochenes, und/oder gegebenenfalls durch =O oder wie zuvor für $R^2$ und $R^3$ in der Bedeutung von Alkyl substituiertes, und/oder mit unsubstituiertem oder wie zuvor für die Arylreste genannt substituiertem Benzol, Furan, Thiophen oder Pyrrol kondensiertes Alkylen mit 2 bis 5 C-Atomen sind, oder

$R^2$ die zuvor angegebene Bedeutung hat und $R^1$ und $R^3$ zusammen gegebenenfalls durch 1 oder 2 -O-, -S-

oder $-NR^4-$ unterbrochenes, und/oder gegebenenfalls durch =O oder wie zuvor für $R^2$ und $R^3$ in der Bedeutung von Alkyl substituiertes, und/oder mit unsubstituiertem oder wie zuvor definiert substituiertem Benzol, Furan, Thiophen oder Pyrrol kondensiertes Alkylen mit 2 bis 5 C-Atomen sind, und * für überwiegend R- oder S-Konfiguration steht, durch asymmetrisch katalysierte Hydrierung von prochiralen Ketiminen der Formel II

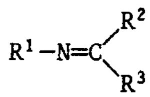

(II),

worin $R^1$, $R^2$ und $R^3$ die zuvor angegebenen Bedeutungen haben, in Gegenwart von Komplexsalzen eines Edelmetalls mit chiralen Liganden, das dadurch gekennzeichnet ist, dass man die Hydrierung bei einer Temperatur von -20 bis 80°C und einem Wasserstoffdruck von $10^5$ Pa bis $10^7$ Pa vornimmt und dem Reaktionsgemisch katalytische Mengen einer Iridiumverbindung der Formel III oder IIIa

[XIrYZ] (III) oder $[XIrY]^{\oplus}A^{\ominus}$ (IIIa)

zugibt, worin X für zwei Olefinliganden oder einen Dienliganden steht, Y ein chirales Diphosphin, dessen sekundäre Phosphingruppen durch 2-4 C-Atome verknüpft sind und das zusammen mit dem Ir-Atom einen 5-, 6- oder 7-Ring bildet, oder Y ein chirales Diphosphinit, dessen Phosphinitgruppen über 2 C-Atome verknüpft sind und das zusammen mit dem Ir-Atom einen 7-Ring bildet, bedeutet, Z für Cl, Br oder J steht, und $A^{\ominus}$ das Anion einer Sauerstoff- oder Komplexsäure darstellt.

$R^1$ kann in beliebigen Stellungen durch gleiche oder verschiedene Reste substituiert sein, z.B. mit 1 bis 5, vorzugsweise 1 bis 3 Substituenten.

Geeignete Substituenten für $R^1$ in der Bedeutung von $C_7$-$C_{16}$-Aralkyl sowie $R^2$ und $R^3$ in der Bedeutung von $C_6$-$C_{12}$-Aryl und $C_7$-$C_{16}$-Aralkyl sind: $C_1$-$C_{12}$-, bevorzugt $C_1$-$C_6$- und besonders $C_1$-$C_4$-Alkyl, -Alkoxy oder -Alkylthio, z.B. Methyl, Ethyl, Propyl, n-, i- und t-Butyl, die Isomeren von Pentyl, Hexyl, Octyl, Nonyl, Decyl, Undecyl und Dodecyl, sowie entsprechende Alkoxy- und Alkylthioreste;

$C_1$-$C_6$-, vorzugsweise $C_1$-$C_4$-Haloalkyl mit vorzugsweise F und Cl als Halogen, z.B. Trifluor- oder Trichlormethyl, Difluorchlormethyl, Fluordichlormethyl, 1,1-Difluoreth-1-yl, 1,1-Dichloreth-1-yl, 1,1,1-Trichlor- oder -Trifluoreth-2-yl, Pentachlorethyl, Pentafluorethyl, 1,1,1-Trifluor-2,2-dichlorethyl, n-Perfluorpropyl, i-Perfluorpropyl, n-Perfluorbutyl, Fluor- oder Chlormethyl, Difluor- oder Dichlormethyl, 1-Fluor- oder -Chlor-eth-2-yl oder -eth-1-yl, 1-, 2-oder 3-Fluor- oder -Chlor-prop-1-yl oder -prop-2-yl oder -prop-3-yl, 1-Fluor- oder -Chlor-but-1-yl, -but-2-yl, -but-3-yl oder -but-4-yl, 2,3-Dichlor-prop-1-yl, 1-Chlor-2-fluor-prop-3-yl, 2,3-Dichlorbut-1-yl;

Halogen, bevorzugt F und Cl;

$C_6$-$C_{12}$-Aryl, -Aryloxy oder -Arylthio, in denen Aryl bevorzugt für Naphthyl und besonders Phenyl steht,

$C_7$-$C_{16}$-Aralkyl, -Aralkoxy und -Aralkylthio, in denen der Arylrest bevorzugt Naphthyl und besonders Phenyl ist und der Alkylenrest linear oder verzweigt ist und 1 bis 10, vorzugsweise 1 bis 6 und insbesondere 1-3 C-Atome enthält, z.B. Benzyl, Naphthylmethyl, 1- oder 2-Phenyleth-1-yl oder -eth-2-yl, 1-, 2- oder 3-Phenyl-prop-1-yl, -prop-2-yl oder -prop-3-yl, besonders bevorzugt ist Benzyl;

die zuvor genannten Arylgruppen enthaltenden Reste können ihrerseits ein-oder mehrfach substituiert sein, z.B. durch $C_1$-$C_4$-Alkyl, -Alkoxy oder -Alkylthio, Halogen, -OH, -CN, $-CONR^4R^5$ oder $-COOR^4$, wobei $R^4$ und $R^5$ die angegebenen Bedeutungen haben; Beispiele sind Methyl, Ethyl, n- und i-Propyl, Butyl, entsprechende Alkoxy- und Alkylthioreste, F, Cl, Br, Dimethyl-, Methylethyl-, Diethylcarbamoyl, Methoxy-, Ethoxy-, Phenoxy- und Benzyloxycarbonyl,

Sekundäramino mit 2 bis 24, vorzugsweise 2 bis 12 und besonders 2 bis 6 C-Atomen, wobei das Sekundäramino bevorzugt 2 Alkylgruppen enthält, z.B. Dimethyl-, Methylethyl-, Diethyl-, Methyl-n-propyl-, Methyl-n-butyl-, Di-n-Propyl-, Di-n-Butyl-, Di-n-Hexylamino;

$-CONR^4R^5$, worin $R^4$ und $R^5$ unabhängig voneinander $C_1$-$C_{12}$-, vorzugsweise $C_1$-$C_6$- und insbesondere $C_1$-$C_4$-Alkyl oder $R^4$ und $R^5$ zusammen Tetra- oder Pentamethylen oder 3-Oxapentylen sind, wobei das Alkyl linear oder verzweigt sein kann, z.B. Dimethyl-, Methylethyl-, Diethyl-, Methyl-n-propyl-, Ethyl-n-propyl-, Di-n-propyl-, Methyl-n-butyl-, Ethyl-n-butyl-, n-Propyl-n-butyl- und Di-n-butylcarbamoyl;

$-COOR^4$, worin $R^4$ $C_1$-$C_{12}$-, bevorzugt $C_1$-$C_6$-Alkyl ist, das linear oder verzweigt sein kann, z.B. Methyl, Ethyl, n- und i-Propyl, n-, i- und t-Butyl, und die Isomeren von Pentyl, Hexyl, Heptyl, Octyl, Nonyl, Decyl, Undecyl und Dodecyl.

$R^1$ als mit Heteroaryl substituiertes Alkyl leitet sich bevorzugt von einem 5- oder 6-gliedrigen Ring mit 1 oder 2 gleichen oder verschiedenen Heteroatomen, besonders O, S oder N ab, das bevorzugt 4 oder 5 C-Atome enthält und das mit Benzol kondensiert sein kann. Beispiele für Heteroaromaten sind Furan, Pyrrol, Thiophen, Pyridin, Pyrimidin, Indol und Chinolin.

$R^1$ als Heterocycloalkyl oder als mit Heterocycloalkyl substituiertes Alkyl enthält bevorzugt 4 bis 6 Ringatome und 1 oder 2 gleiche oder verschiedene Heteroatome aus der Gruppe O, S und $NR^4$. Es kann mit Benzol kondensiert sein. Es kann sich z.B. von Pyrrolidin, Tetrahydrofuran, Tetrahydrothiophen, Indan, Pyrazolidin, Oxazolidin, Piperidin, Piperazin oder Morpholin ableiten.

Für die Substituenten von $R^2$ und $R^3$ gelten die gleichen Bevorzugungen wie für die Substituenten von $R^1$.

$R^1$, $R^2$ und $R^3$ als Alkyl sind bevorzugt unsubstituiertes oder substituiertes $C_1$-$C_6$-, besonders $C_1$-$C_4$-Alkyl, das linear oder verzweigt sein kann. Beispiele sind Methyl, Ethyl, i- und n-Propyl, i-, n- und t-Butyl, die Isomeren von Pentyl, Hexyl, Heptyl, Octyl, Nonyl, Decyl, Undecyl und Dodecyl.

$R^1$, $R^2$ und $R^3$ als unsubstituiertes oder substituiertes Cycloalkyl enthalten bevorzugt 3 bis 6, besonders 5 oder 6 Ring-C-Atome. Beispiele sind Cyclopropyl, Cyclobutyl, Cyclopentyl, Cyclohexyl, Cycloheptyl und Cyclooctyl.

$R^2$ und $R^3$ als Aryl sind bevorzugt unsubstituiertes oder substituiertes Naphthyl und besonders Phenyl.

$R^1$, $R^2$ und $R^3$ als Aralkyl sind bevorzugt unsubstituiertes oder substituiertes Phenylalkyl mit 1-10, bevorzugt 1 bis 6 und besonders 1 bis 4 C-Atomen im Alkylen, wobei das Alkylen linear oder verzweigt sein kann. Beispiele sind besonders Benzyl, sowie 1-Phenyleth-1-yl, 2-Phenyleth-1-yl, 1-Phenylprop-1-yl, 1-Phenylprop-2-yl, 1-Phenylprop-3-yl, 2-Phenylprop-1-yl, 2-Phenylprop-2-yl und 1-Phenylbut-4-yl.

In $R^2$ und $R^3$ als $-CONR^4R^5$ und $-COOR^4$ stellen $R^4$ und $R^5$ bevorzugt $C_1$-$C_6$-, besonders $C_1$-$C_4$-Alkyl oder $R^4$ und $R^5$ zusammen Tetramethylen, Pentamethylen oder 3-Oxapentylen dar. Beispiele für Alkyl sind zuvor erwähnt worden.

$R^2$ und $R^3$ zusammen oder $R^1$ und $R^3$ zusammen als Alkylen sind bevorzugt durch 1 -O-, -S- oder $-NR^4$-, vorzugsweise -O-, unterbrochen. $R^2$ und $R^3$ zusammen bzw. $R^1$ und $R^3$ zusammen bilden mit dem C-Atom bzw. mit der -N=C-Gruppe, an das sie gebunden sind, bevorzugt einen 5-oder 6-gliedrigen Ring. Für die Substituenten gelten die zuvor erwähnten Bevorzugungen, wobei zusätzlich $C_1$-$C_4$-Alkyl als Substituent in Frage kommt. Als kondensiertes Alkylen sind $R^2$ und $R^3$ zusammen bzw. $R^1$ und $R^3$ zusammen bevorzugt mit Benzol oder Pyridin kondensiertes Alkylen. Beispiele für Alkylen sind: Ethylen, 1,2-oder 1,3-Propylen, 1,2-, 1,3- oder 1,4-Butylen, 1,5-Pentylen und 1,6-Hexylen. Beispiele für unterbrochenes oder durch =O substituiertes Alkylen sind 2-Oxa-1,3-propylen, 2-Oxa-1,4-butylen, 2-Oxa- oder 3-Oxa-1,5-pentylen, 3-Thia-1,5-pentylen, 2-Thia-1,4-butylen, 2-Thia-1,3-propylen, 2-Methylimino-1,3-propylen, 2-Ethylimino-1,4-butylen, 2- oder 3-Methylimino-1,5-pentylen, 1-Oxo-2-oxa-1,3-propylen, 1-Oxo-2-oxa-1,4-butylen, 2-Oxo-3-oxa-1,4-butylen, 1-Oxa-2-oxo-1,5-pentylen. Beispiele für kondensiertes Alkylen sind:

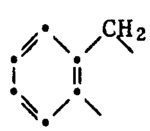 , 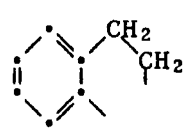 , 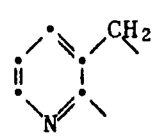 und 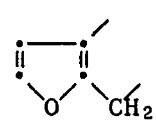 .

Beispiele für kondensiertes und unterbrochenes und gegebenenfalls mit =O substituiertes Alkylen sind

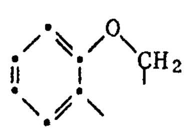 , 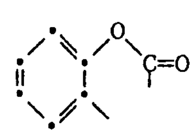 und 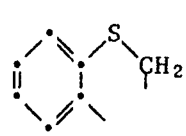 .

In einer bevorzugten Gruppe steht $R^1$ für (Methoxycarbonyl)-methyl und $R^2$ und $R^3$ zusammen für den Rest der Formel

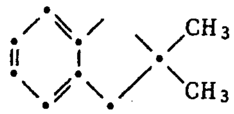 .

Imine der Formel II sind bekannt oder sie können nach bekannten Verfahren aus Ketonen und entsprechenden Aminen hergestellt werden. In einer Ausführungsform des Verfahrens können die Imine der Formel II auch in situ aus den entsprechenden Ketonen und Aminen hergestellt werden.

Das Verfahren wird bevorzugt bei einer Temperatur von -20 bis 50 °C, besonders -20 bis 30 °C, insbesondere -20 bis 20 °C, und bevorzugt bei einem Wasserstoffdruck von $2 \cdot 10^5$ bis $6 \cdot 10^6$ Pa, besonders $8 \cdot 10^5$ bis $6 \cdot 10^6$ Pa durchgeführt.

In den Formeln III und IIIa kann X als Olefinligand z.B. Buten, Propen und besonders Ethylen sein und der Dienligand ist bevorzugt ein offenkettiges oder cyclisches Dien, dessen Doppelbindungen durch ein oder zwei C-Atome verknüpft sind. Das Dien ist bevorzugt Hexadien, Cyclooctadien oder Norbornadien.

Im chiralen Diphosphin sind die Phosphingruppen bevorzugt über eine aliphatische Gruppe mit 2-4 C-Atomen verknüpft, die durch $C_1$-$C_4$-Alkyl, $C_5$- oder $C_6$-Cycloalkyl, Phenyl oder Benzyl substituiert sein kann. Bei der aliphatischen Gruppe kann es sich um Alkylen oder um eine cycloaliphatische Gruppe mit 5 oder 6 Ring-C-Atomen oder um eine aliphatischheterocyclische Gruppe mit 1 bis 2 -O- oder =N-$C_1$-$C_{12}$-Alkyl, oder -Acyl oder -Aminocarbonyl, -Phenyl oder -Benzyl und 3-5 C-Atomen im Ring handeln. Die Ringe können durch $C_1$-$C_4$-Alkyl, $C_5$-$C_7$-Cycloalkyl, Phenyl oder Benzyl substituiert sein.

Y in den Formeln III oder IIIa ist bevorzugt ein chirales Diphosphin, dessen Phosphingruppen durch 4 C-Atome verknüpft sind und das zusammen mit dem Ir-Atom einen 7-Ring bildet.

Die Phosphingruppen und Phosphinitgruppen enthalten bevorzugt $C_1$-$C_{12}$-Alkyl, Cycloalkyl mit 5 bis 8 Ring-C-Atomen, das durch 1 bis 3 $C_1$-$C_6$-Alkylgruppen substituiert sein kann, Phenyl, $C_7$-$C_{12}$-Phenylalkyl oder Alkylphenylalkyl mit 1 bis 6 C-Atomen in den Alkylgruppen und 1 bis 5 C-Atomen in der Alkylengruppe. Besonders bevorzugt sind t-Butyl, Phenyl, Benzyl oder Cyclohexyl. Geeignete chirale Diphosphine sind beschrieben in H.B. Kagan, Chiral Ligands for Asymmetric Catalysis, Asymmetric Synthesis, Volume 5, S. 13-23, Academic Press, Inc., N.Y. (1985).

Beispiele sind (Ph steht für Phenyl):

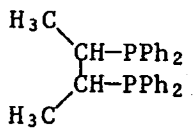 , 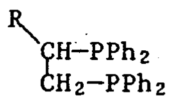 R = Methyl, Phenyl, Cyclohexyl,

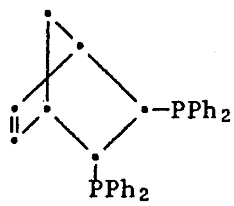 , 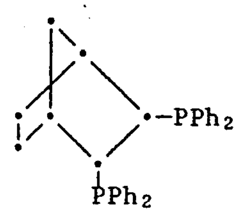 ,

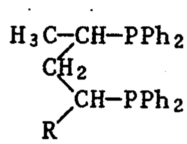 R=H,$CH_3$ , 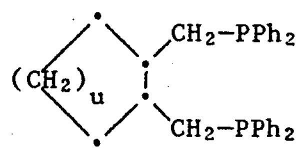 u=0, 1, 2 ,

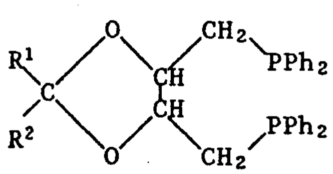 $R^1$=H,$CH_3$,Ph, $R^2$=H,$CH_3$,Ph 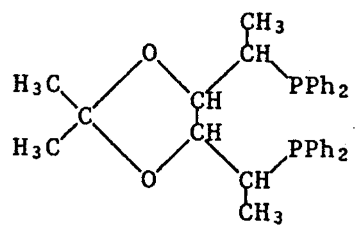 ,

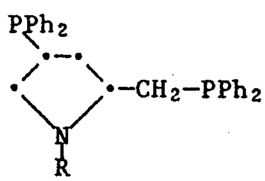 R=-$CO_2$-t-Butyl, -CO-t-Butyl, -CONH$C_1$-$C_4$-Alkyl,

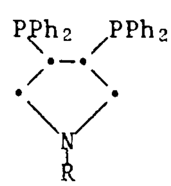 R=Benzyl, $C_1$-$C_4$-Alkyl.

Ein Beispiel für Disphosphinite ist 1-O-Phenyl-4,6,-O-(R)-benzyliden-2,3-O-bis(diphenylphosphino)-β-D-glucopyranosid der Formel

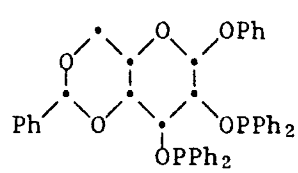 .

In Formel III steht Z bevorzugt für Cl oder Br. $A^\ominus$ in Formel IIIa steht bevorzugt für $ClO_4^\ominus$, $CF_3SO_3^\ominus$, $BF_4^\ominus$, $B(Phenyl)_4^\ominus$, $PF_6^\ominus$, $SbCl_6^\ominus$, $AsF_6^\ominus$ oder $SbF_6^\ominus$.

Eine bevorzugte Gruppe von Iridiumverbindungen sind solche der Formel III, worin X für Cyclooctadien, Z für Cl und Y für chirale Diphosphine stehen.

Die Iridiumverbindungen der Formeln III und IIIa sind bekannt oder können nach bekannten Verfahren hergestellt werden, siehe z.B. R. Uson et al., Inorg. Chim. Acta 73, S. 275 ff (1983); S. Brunie et al., Journal of Organometallic Chemistry, 114 (1976), S. 225-235 und M. Green et al., J. Chem. Soc. (A), S. 2334 ff (1971).

Die Iridiumverbindungen können als isolierte Verbindungen eingesetzt werden. Es ist zweckmässig, die Verbindungen in situ herzustellen und direkt zu verwenden.

Die Iridiumverbindungen werden bevorzugt in Mengen von 0.01 bis 5, besonders 0,05 bis 2 Mol-% eingesetzt, bezogen auf die Verbindungen der Formel II. Das Molverhältnis von Verbindung der Formel II zu Verbindung der Formel III bzw. IIIa beträgt bevorzugt 500 bis 40, besonders 200 bis 50.

Eine bevorzugte Verfahrensdurchführung ist dadurch gekennzeichnet, dass man zusätzlich ein Ammonium- oder Alkalimetallchlorid, -bromid oder -jodid verwendet. Der Zusatz von Chloriden, Bromiden oder Jodiden ist besonders dann zweckmässig, wenn man Verbindungen der Formel IIIa als Katalysatoren einsetzt. Die Chloride, Bromide und Jodide können beispielsweise in Mengen von 0,1 bis 100, bevorzugt aber von 1 bis 50 und ganz bevorzugt von 2 bis 20 Aequivalenten, bezogen auf die Verbindungen der Formel III oder IIIa, eingesetzt werden. Als Salze sind die Jodide bevorzugt. Ammonium ist bevorzugt Tetraalkylammonium mit 1 bis 6 C-Atomen in den Alkylgruppen und als Alkalimetall ist Natrium, Lithium oder Kalium bevorzugt.

Die Reaktion kann ohne oder in Gegenwart von Lösungsmitteln durchgeführt werden. Geeignete Lösungsmittel, die alleine oder als Mischung von Lösungsmitteln eingesetzt werden können, sind zum Beispiel:

Aliphatische und aromatische Kohlenwasserstoffe, wie z.B. Pentan, Hexan, Cyclohexan, Methylcyclohexan, Benzol, Toluol und Xylol; Alkohole, wie z.B. Methanol, Ethanol, Propanol und Butanol; Ether, wie z.B. Diethylether, Diethylenglykoldimethylether, Tetrahydrofuran und Dioxan; Halogenkohlenwasserstoffe, wie z.B. Methylenchlorid, Chloroform, 1,1,2,2-Tetrachlorethan und Chlorbenzol; Ester und Lactone, wie z.B. Essigsäureethylester, Butyrolacton und Valerolacton; N-substituierte Säureamide und Lactame, wie z.B. Dimethylformamid, Dimethylacetamid und N-Methylpyrrolidon. Vorteilhaft sind Mischungen aus einem aromatischen Kohlenwasserstoff und Alkoholen, z.B. Toluol/Ethanol oder Benzol/Methanol.

Die Verbindungen der Formel I sind Zwischenprodukte zur Herstellung biologisch aktiver Substanzen, insbesondere im Bereich der Pharmazeutika und Agrochemikalien. So können aus den sekundären Aminen, besonders N-Carbalkoxymethylaminen, 5-Imidazolcarbonsäurederivate hergestellt werden, die eine herbizide Wirkung haben und zur Bekämpfung von Unkräutern verwendet werden können (vgl. EP-A-0 207 563).

Die nachfolgenden Beispiele erläutern die Erfindung näher.

Beispiel 1:

In einen 250 ml Zweihalskolben werden unter Argon-Schutzgas 10 mmol Ketimin eingetragen. Der Kolben wird dreimal evakuiert und mit Argon gespült. Danach werden je 5 ml Methanol und Benzol zugegeben und 2 Minuten bei Raumtemperatur gerührt (Lösung A).

In einen 25 ml Zweihalskolben werden unter Argon-Schutzgas je 5 ml Methanol und Benzol eingetragen. Danach werden nacheinander 0,1 mmol $[Ir(Cyclooctadien\text{-})Cl]_2$, 0,22 mmol Diphosphin und wo angegeben 0,4 mmol Tetrabutylammoniumjodid (TBAI) zugefügt. Nach jeder Zugabe wird so lange gerührt, bis eine homogene Lösung vorliegt (Lösung B).

In einen 120 ml Glasautoklaven oder 65 ml Stahlautoklaven werden unter Luftausschluss die Lösungen A und B nacheinander eingetragen. Durch ein Gaseinlassventil werden $2 \cdot 10^6$ Pa Wasserstoff aufgepresst. Gleichzeitig werden in ein 100 ml Reservoir $1{,}4 \cdot 10^7$ Pa Wasserstoff aufgepresst. Die Temperatur beträgt 20 - 22 °C. Die Reaktion wird solange bei einem konstanten Wasserstoffdruck von $2 \cdot 10^6$ Pa durchgeführt, bis keine Wasserstoffaufnahme mehr erfolgt. Danach wird das Reaktionsgemisch mit Stickstoff in einen 250 ml Kolben gespült. Das Lösungsmittel wird bei 80 °C am Rotationsverdampfer entfernt. Man erhält ein Rohprodukt, das man im Hochvakuum (1-10 Pa) destilliert. Anschliessend bestimmt man polarometrisch die optische Ausbeute (A.F. Lee et al., J. Chem. Arc. 1954, 145), oder mittels $^1$H-HMR unter Verwendung von Shiftreagenzien, oder mittels HPLC unter Verwendung einer Säule mit (R)-Dinitrobenzoylphenylglycin Covalent.

Die optischen Ausbeuten (ee in %), Reaktionszeiten und der Umsatz sind in Tabelle 1 angegeben.

Tabelle 1

| Ketimin | Umsatz (%) | Reaktionszeit (Stunden) | Optische Ausbeute (% ee) |
|---|---|---|---|
| 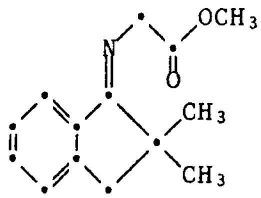 | 99 | 1,8 | 23,4 |
| 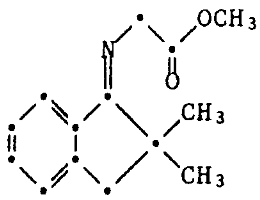 (*) | 99 | 22 | 23,4 |
| 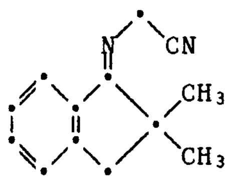 | 53 | 75 | 26,4 |
| 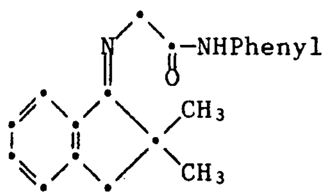 | 98 | 7 | 10 |
| 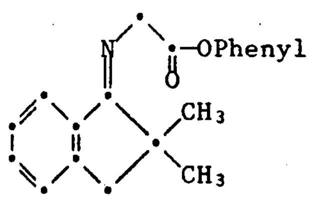 | 99 | 7 | 23 |
| 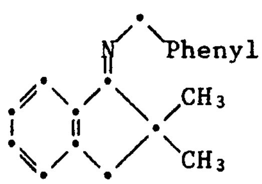 | 40 | 22,5 | 26 |

(*) 20 mMol

Tabelle 1 (Fortsetzung)

| Ketimin | Umsatz (%) | Reaktionszeit (Stunden) | Optische Ausbeute (% ee) |
|---|---|---|---|
| 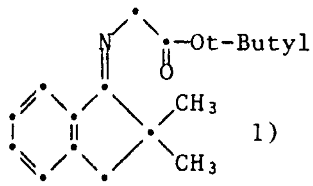 1) | 99 | 2,3 | 16,5 |
| 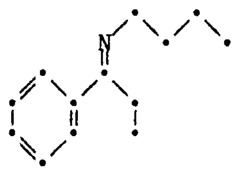 | 99 | 1 | 16 |
| 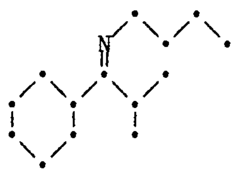 | 99 | 0,5 | 18 |
| 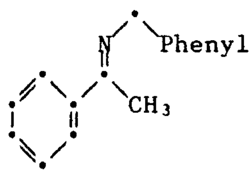 | 99 | 5 | 30 |
| 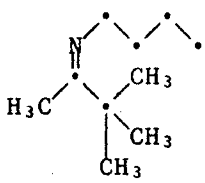 | 99 | 0,5 | 8 |
| 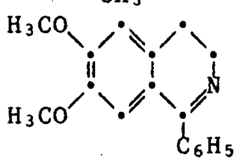 | 38 | 21,5 | 5 |
| 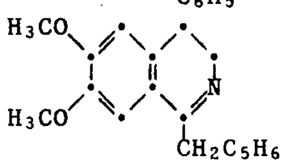 | 96 | 69 | 29 |
| 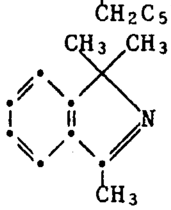 | 40 | 22 | 15 |

1) 6,5 mmol

Katalysator; [Ir(Cyclooctadien)Cl]$_2$ und (-)-DIOP

Zusatz: 2-Aequivalente TBAI, bezogen auf Katalysator

Bestimmung % ee: mittels HPLC oder $^1$H-NMR-Spektroskopie

Beispiel 2:

Es wird wie in Beispiel 1 verfahren und verschiedene Diphosphine eingesetzt. Als Ketimin wird verwendet:

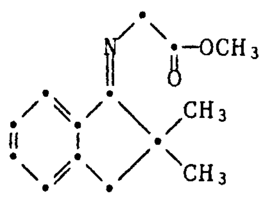 .

Die Ergebnisse sind in Tabelle 2 angegeben.

Tabelle 2

| Diphosphin | Umsatz (%) | Reaktionszeit (Stunden) | Optische Ausbeute (% ee) | Konfiguration |
|---|---|---|---|---|
| 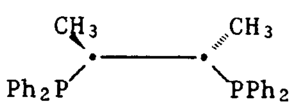 (CHIRAPHOS) | 81 | 65 | 16 | S |
| 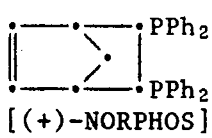 [(+)-NORPHOS] | 86 | 22,5 | 4 | R |
| 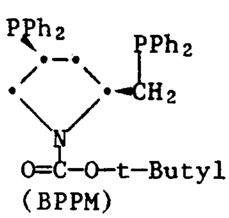 (BPPM) | 95 | 0,4 | 29,5 | R |

Tabelle 2 (Fortsetzung)

| Diphosphin | Umsatz | Reaktionszeit (Stunden) | Optische Ausbeute (% ee) | Konfiguration |
|---|---|---|---|---|
| 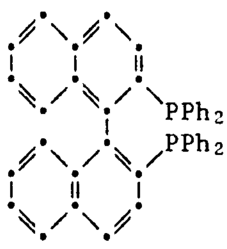 [(-)-BINAP] | 83 | 17,5 | 19,6 | S |
| 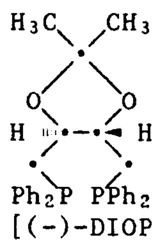 [(-)-DIOP] | 99 | 1,8 | 23,4 | R |
| 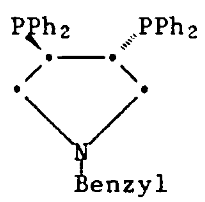 | 99 | 5 | 24 | S |

**Molverhältnis Ketimin : Katalysator = 50 : 1**

**Katalysator: [Ir (Cyclooctadien)Cl]$_2$ und 2,2 Aequivalente Diphosphin**

**Zusatz: 2 Aequivalente TBAI, bezogen auf Katalysator**

**Bestimmung % ee: Mittels HPLC**

Beispiel 3:

Es wird wie in Beispiel 1 verfahren und die Reaktionsbedingungen variiert. Die Ergebnisse sind in Tabelle 3 zusammengefasst.

Tabelle 3

| Zusatz (Aequivalente TBAI) | Lösungsmittel | Druck ($10^6$ Pa) | Temperatur (°C) | Umsatz (%) | Reaktionszeit (Stunden) | Optische Ausbeute (% ee) | Konfiguration |
|---|---|---|---|---|---|---|---|
| 2 | Methanol/Benzol (1:1) | 20 | 20 | 99 | 1 | 23,4 | R |
| 10 | " | 20 | 20 | 99 | 2 | 22,0 | R |
| 2 | " | 20 | 50 | 99 | 0,5 | 21,7 | R |
| 2 | " | 20 | 0 | 99 | 4 | 25,0 | R |
| 2 | " | 10 | 20 | 99 | 6 | 23,0 | R |
| 2 | " | 50 | 20 | 99 | 1 | 22,6 | R |
| 2 | Tetrahydrofuran | 20 | 20 | 99 | 4 | 19 | R |
| - | " | 20 | 20 | 92 | 22 | 3,5 | S |
| - | Methanol | 20 | 20 | 62 | 22,5 | 2 | R |
| - | Methylenchlorid | 20 | 20 | 97 | 20 | 9 | S |
| 2 | Dimethylformamid | 20 | 20 | 91 | 18 | 8,5 | R |
| - | " | 20 | 20 | 99 | 21 | 4 | S |

Ketimin gemäss Beispiel 2

Katalysator gemäss Beispiel 1

Molverhältnis Ketimin : Katalysator = 50

## Patentansprüche

1. Verfahren zur Herstellung von optisch aktiven sekundären N-aliphatischen Aminen der Formel I

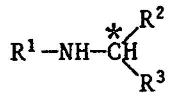 (I),

worin $R^1$ lineares oder verzweigtes $C_1$-$C_{12}$-Alkyl, Cycloalkyl mit 3 bis 8 Ring-C-Atomen, über ein C-Atom gebundenes Heterocycloalkyl mit 3 bis 8 Ringatomen und 1 oder 2 Heteroatomen aus der Gruppe O, S und $NR^4$, $C_7$-$C_{16}$-Aralkyl oder mit dem genannten Cycloalkyl oder Heterocycloalkyl oder Heteroaryl substituiertes $C_1$-$C_{12}$-Alkyl bedeutet, die unsubstituiert oder mit $C_1$-$C_{12}$-Alkyl, $C_1$-$C_{12}$-Alkoxy, $C_1$-$C_{12}$-Alkylthio, $C_1$-$C_6$-Haloalkyl, Halogen, -OH, -CN, $C_6$-$C_{12}$-Aryl oder -Aryloxy oder -Arylthio, $C_7$-$C_{16}$-Aralkyl oder -Aralkoxy oder -Aralkylthio, wobei die Arylreste ihrerseits durch $C_1$-$C_4$-Alkyl, -Alkoxy, -Alkylthio, Halogen, -OH, -CN, $-CONR^4R^5$ oder $-COOR^4$ substituiert sein können, Sekundäramino mit 2 bis 24 C-Atomen,

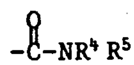

oder

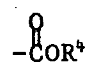 ,

wobei $R^5$ für H steht oder unabhängig die Bedeutung von $R^4$ hat und $R^4$ $C_1$-$C_{12}$-Alkyl, Phenyl oder Benzyl oder $R^4$ und $R^5$ zusammen Tetra- oder Pentamethylen oder 3-Oxapentylen sind, substituiert sein können;
$R^2$ und $R^3$ voneinander verschieden sind und gegebenenfalls mit -OH, -CN, Halogen, $C_1$-$C_{12}$-Alkoxy, Phenoxy, Benzyloxy, Sekundäramino mit 2 bis 24 C-Atomen,

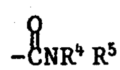

oder $-COOR^4$ substituiertes $C_1$-$C_{12}$-Alkyl oder Cycloalkyl mit 3-8 Ring-C-Atomen, gegebenenfalls wie zuvor für $R^1$ in der Bedeutung von Aralkyl und Aryl substituiertes $C_6$-$C_{12}$-Aryl oder $C_7$-$C_{16}$-Aralkyl oder $-CONR^4R^5$ oder $-COOR^4$ darstellen, worin $R^4$ und $R^5$ die zuvor angegebene Bedeutung haben; oder
$R^1$ die zuvor angegebene Bedeutung hat und $R^2$ und $R^3$ zusammen gegebenenfalls durch 1 oder 2 -O-, -S- oder $-NR^4$- unterbrochenes, und/oder gegebenenfalls durch =O oder wie zuvor für $R^2$ und $R^3$ in der Bedeutung von Alkyl substituiertes, und/oder mit unsubstituiertem oder wie zuvor für die Arylreste genannt substituiertem Benzol, Furan, Thiophen oder Pyrrol kondensiertes Alkylen mit 2 bis 5 C-Atomen sind, oder
$R^2$ die zuvor angegebene Bedeutung hat und $R^1$ und $R^3$ zusammen gegebenenfalls durch 1 oder 2 -O-, -S- oder $-NR^4$- unterbrochenes, und/oder gegebenenfalls durch =O oder wie zuvor für $R^2$ und $R^3$ in der Bedeutung von Alkyl substituiertes, und/oder mit unsubstituiertem oder wie zuvor definiert substituiertem Benzol, Furan, Thiophen oder Pyrrol kondensiertes Alkylen mit 2 bis 5 C-Atomen sind, und * für überwiegend R- oder S-Konfiguration steht, durch asymmetrisch katalysierte Hydrierung von prochiralen Ketiminen der Formel II

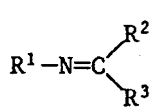 (II),

worin $R^1$, $R^2$ und $R^3$ die zuvor angegebenen Bedeutungen haben, in Gegenwart vom Komplexsalzen eines Edelmetalls mit chiralen Liganden, dadurch gekennzeichnet, dass man die Hydrierung bei einer Temperatur von -20 bis 80 °C und einem Wasserstoffdruck von $10^5$ Pa bis $10^7$ Pa vornimmt und dem Reaktionsgemisch katalytische Mengen einer Iridiumverbindung der Formel III oder IIIa

[XIrYZ] (III) oder $[XIrY]^{\oplus}A^{\ominus}$ (IIIa)

zugibt, worin X für zwei Olefinliganden oder einen Dienliganden steht, Y ein chirales Diphosphin, dessen sekundäre Phosphingruppen durch 2-4 C-Atome verknüpft sind und das zusammen mit dem Ir-Atom einen 5-, 6- oder 7-Ring bildet, oder Y ein chirales Diphosphinit, dessen Phosphinitgruppen über 2 C-Atome verknüpft sind und das zusammen mit dem Ir-Atom einen 7-Ring bildet, beduetet, Z für Cl, Br oder J steht, und $A^{\ominus}$ das Anion einer Sauerstoff- oder Komplexsäure darstellt.

**2.** Verfahren gemäss Anspruch 1, dadurch gekennzeichnet, dass die Reaktionstemperatur -20 bis 50 °C beträgt.

**3.** Verfahren gemäss Anspruch 1, dadurch gekennzeichnet, dass der Wasserstoffdruck $2 \cdot 10^5$ Pa bis $6 \cdot 10^6$ Pa beträgt.

**4.** Verfahren gemäss Anspruch 1, dadurch gekennzeichnet, dass X in den Formeln III und IIIa zwei Ethylen oder ein offenkettiges oder cyclisches Dien ist, dessen Doppelbindungen über 1 oder 2 C-Atome verknüpft sind.

**5.** Verfahren gemäss Anspruch 4, dadurch gekennzeichnet, dass das Dien Hexadien, Norbornadien oder Cyclooctadien ist.

**6.** Verfahren gemäss Anspruch 1, dadurch gekennzeichnet, dass Y in den Formeln III und IIIa ein chirales Diphosphin ist, dessen Phosphingruppen durch 4 C-Atome verknüpft sind und das zusammen mit dem Ir-Atom einen 7-Ring bildet.

**7.** Verfahren gemäss Anspruch 1, dadurch gekennzeichnet, dass die Phosphin- und Phosphinitgruppen $C_1$-$C_{12}$-Alkyl, Cycloalkyl mit 5 bis 8 Ring-C-Atomen, das durch 1 bis 3 $C_1$-$C_6$-Alkylgruppen substituiert sein kann, Phenyl, $C_7$-$C_{12}$-Phenylalkyl oder Alkylphenylalkyl mit 1 bis 6 C-Atomen in den Alkylgruppen und 1 bis 5 C-Atomen in der Alkylengruppe enthalten.

**8.** Verfahren gemäss Anspruch 1, dadurch gekennzeichnet, dass $A^{\ominus}$ für $ClO_4^{\ominus}$, $CF_3SO_3^{\ominus}$, $BF_4^{\ominus}$, B-$(Phenyl)_4^{\ominus}$, $PF_6^{\ominus}$, $SbCl_6^{\ominus}$, $AsF_6^{\ominus}$ oder $SbF_6^{\ominus}$ steht.

**9.** Verfahren gemäss Anspruch 1, dadurch gekennzeichnet, dass die Iridiumverbindung in einer Menge von 0,01 bis 5 Mol-% zugegeben wird, bezogen auf die Verbindung der Formel II.

**10.** Verfahren gemäss Anspruch 1, dadurch gekennzeichnet, dass man zusätzlich ein Ammonium- oder Alkalimetallchlorid, -bromid oder -jodid zugibt.

**11.** Verfahren gemäss Anspruch 1, dadurch gekennzeichnet, dass in Formel III X für Cyclooctadien, Z für Cl und Y für ein chirales Diphosphin stehen.

**Claims**

**1.** A process for the preparation of optically active secondary N-aliphatic amines of the formula I

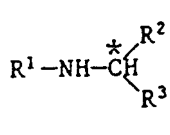 (I),

in which $R^1$ is linear or branched $C_1$-$C_{12}$alkyl, cycloalkyl having 3 to 8 ring C atoms, heterocycloalkyl which is linked via a C atom and has 3 to 8 ring atoms and 1 or 2 heteroatoms selected from the group

consisting of O, S and $NR^4$, $C_7$-$C_{16}$aralkyl or $C_1$-$C_{12}$alkyl which is substituted by the cycloalkyl or heterocycloalkyl or heteroaryl mentioned, each of which can be unsubstituted or substituted by $C_1$-$C_{12}$alkyl, $C_1$-$C_{12}$alkoxy, $C_1$-$C_{12}$alkylthio, $C_1$-$C_6$halogenoalkyl, halogen, -OH, -CN, $C_6$-$C_{12}$aryl, $C_6$-$C_{12}$aryloxy, $C_6$-$C_{12}$arylthio, $C_7$-$C_{16}$aralkyl, $C_7$-$C_{16}$aralkoxy or $C_7$-$C_{16}$aralkylthio, it being possible for the aryl radicals in turn to be substituted by $C_1$-$C_4$alkyl, $C_1$-$C_4$alkoxy, $C_1$-$C_4$alkylthio, halogen, -OH, -CN, $-CONR^4R^5$ or $-COOR^4$, secondary amino having 2 to 24 C atoms,

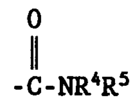

or

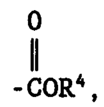

where $R^5$ is H or independently is as defined for $R^4$ and $R^4$ is $C_1$-$C_{12}$alkyl, phenyl or benzyl, or $R^4$ and $R^5$ together are tetramethylene, pentamethylene or 3-oxapentylene; $R^2$ and $R^3$ are different from one another and are $C_1$-$C_{12}$alkyl which is unsubstituted or substituted by -OH, -CN, halogen, $C_1$-$C_{12}$alkoxy, phenoxy, benzyloxy, secondary amine having 2 to 24 C atoms,

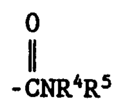

or $-COOR^4$, or are cycloalkyl having 3-8 ring C atoms, $C_6$-$C_{12}$aryl which is unsubstituted or substituted as above for $R^1$ where the latter is aralkyl and aryl, or $C_7$-$C_{16}$aralkyl or $-CONR^4R^5$ or $-COOR^4$ in which $R^4$ and $R^5$ are as defined above; or $R^1$ is as defined above and $R^2$ and $R^3$ together are alkylene which has 2 to 5 C atoms and is, if appropriate, interrupted by 1 or 2 -O-, -S- or $-NR^4$- groups, and/or is unsubstituted or substituted by =O or as above for $R^2$ and $R^3$ when the latter are alkyl, and/or is condensed with benzene, furan, thiophene or pyrrole which is unsubstituted or substituted as mentioned above for the aryl radicals, or $R^2$ is as defined above and $R^1$ and $R^3$ together are alkylene which has 2 to 5 C atoms and is, if appropriate, interrupted by 1 or 2 -O-, -S- or $-NR^4$ groups, and/or is unsubstituted or substituted by =O or as above for $R^2$ and $R^3$ when the latter are alkyl, and/or is condensed with benzene, furan, thiophene or pyrrole which is unsubstituted or substituted as defined above, and * is predominantly R-configuration or S-configuration, by the asymmetric catalysed hydrogenation of prochiral ketimines of the formula II

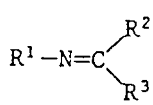
(II)

in which $R^1$, $R^2$ and $R^3$ are as defined above, in the presence of complex salts of a noble metal having chiral ligands, which comprises carrying out the hydrogenation at a temperature from -20 to 80 °C and under a hydrogen pressure from $10^5$ to $10^7$ Pa and adding to the reaction mixture catalytic amounts of an iridium compound of the formula III or IIIa

[XIrYZ] (III) or $[XIrY]^{\oplus}A^{\ominus}$ (IIIa)

in which X is two olefin ligands or a diene ligand, Y is a chiral diphosphine the secondary phosphine groups of which are attached via 2-4 C atoms and which, together with the Ir atom, forms a 5-membered, 6-membered or 7-membered ring, or Y is a chiral diphosphinite the phosphinite groups of

which are attached via 2 C atoms and which, together with the Ir atom, forms a 7-membered ring, Z is Cl, Br or I and $A^{\ominus}$ is the anion of an oxygen acid or complex acid.

**2.** A process according to claim 1, wherein the reaction temperature is -20 to 50 °C.

**3.** A process according to claim 1, wherein the hydrogen pressure is 2 x $10^5$ Pa to 6 x $10^6$ Pa.

**4.** A process according to claim 1, wherein X in the formulae III and IIIa is two ethylene groups or an open-chain or cyclic diene the double bonds of which are attached via 1 or 2 C atoms.

**5.** A process according to claim 4, wherein the diene is hexadiene, norbornadiene or cyclooctadiene.

**6.** A process according to claim 1, wherein Y in the formulae III and IIIa is a chiral diphosphine the phosphine groups of which are attached via 4 C atoms and which, together with the Ir atom, forms a 7-membered ring.

**7.** A process according to claim 1, wherein the phosphine and phosphinite groups contain $C_1$-$C_{12}$alkyl, cycloalkyl which has 5 to 8 ring C atoms and can be substituted by 1 to 3 $C_1$-$C_6$alkyl groups, phenyl, $C_7$-$C_{12}$phenylalkyl or alkylphenylalkyl having 1 to 6 C atoms in the alkyl groups and 1 to 5 C atoms in the alkylene group.

**8.** A process according to claim 1, wherein $A^{\ominus}$ is $ClO_4^{\ominus}$, $CF_3SO_3^{\ominus}$, $BF_4^{\ominus}$, B(phenyl)$_4^{\ominus}$, $PF_6^{\ominus}$, $SbCl_6^{\ominus}$, $AsF_6^{\ominus}$ or $SbF_6^{\ominus}$.

**9.** A process according to claim 1, wherein the iridium compound is added in an amount of 0.01 to 5 mol %, relative to the compound of the formula II.

**10.** A process according to claim 1, wherein, in addition, an ammonium chloride, bromide or iodide or an alkali metal chloride, bromide or iodide is added.

**11.** A process according to claim 1, wherein, in formula III, X is cyclooctadiene, Z is Cl and Y is a chiral diphosphine.

**Revendications**

**1.** Procédé pour préparer des amines N-aliphatiques secondaires optiquement actives, de formule I

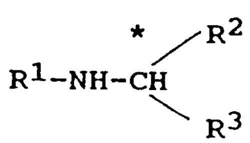

(I),

[dans laquelle $R^1$ représente un groupe alkyle en $C_1$ à $C_{12}$, linéaire ou ramifié, cycloalkyle ayant 3 à 8 atomes de carbone de noyau, un groupe hétérocycloalkyle ayant 3 à 8 atomes de noyau et 1 ou 2 hétéro atomes choisis parmi O, S et $NR^4$, ce groupe étant fixé par l'intermédiaire d'un atome de carbone, un groupe aralkyle en $C_7$ à $C_{16}$,
ou un groupe alkyle en $C_1$ à $C_{12}$, substitué par les restes cycloalkyles ou hétérocycloalkyles précités ou substitué par hétéroaryles, qui ne sont pas substitués ou sont substitués par un groupe alkyle en $C_1$ à $C_{12}$,
alcoxy en $C_1$ à $C_{12}$, alkylthio en $C_1$ à $C_{12}$, halogénoalkyle en $C_1$ à $C_6$, halogéno, -OH, CN, aryle, aryloxy ou arylthio en $C_1$ à $C_{12}$, aralkyle ou aralcoxy ou aralkylthio en $C_7$ à $C_{16}$, les restes aryles pouvant pour leur part être substitués par un reste alkyle, alcoxy, ou alkylthio en $C_1$ à $C_4$ halogéno, -OH, -CN,-$CONR^4R^5$ ou - $COOR^4$, un groupe amino secondaire comportant 2 à 24 atomes de carbone, ou

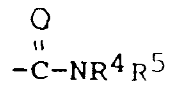

ou -$COOR^4$,$R^5$ représentant H ou ayant, indépendament, le sens de $R^4$, et $R^4$ représentant un groupe alkyle en $C_1$ à $C_{12}$, phényle ou benzyle, ou bien $R^4$ et $R^5$ forment ensemble un groupe tétraméthylène ou pentaméthylène ou 3-oxapentylène;

$R^2$ et $R^3$ sont différents l'un de l'autre et représentent chacun un groupe alkyle en $C_1$ à $C_{12}$ -(éventuellement substitué par -OH, -CN, de l'halogène, un groupe alcoxy en $C_1$ à $C_{12}$, phénoxy, benzyloxy, amino secondaire ayant 2 à 24 atomes de carbone,

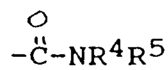

ou -$COOR^4$ ou cycloalkyle comportant 3 à 8 atomes de carbone de noyau, un groupe aryle en $C_6$ à $C_{12}$ ou aralkyle en $C_7$ à $C_{16}$ (éventuellement substitué comme indiqué ci-dessus pour $R^1$ représentant un groupe aralkyle ou aryle) ou -$CONR^4R^5$ ou -$COOR^4$, les symboles $R^4$ et $R^5$ ayant le sens indiqué ci-dessous, ou bien

$R^1$ a le sens indiqué ci-dessus et $R^2$ et $R^3$ forment ensemble un groupe alkylène comportant 2 à 5 atomes de carbone,éventuellement interrompu par 1 ou 2 groupes -O-, -S- ou -$NR^4$- et/ou éventuellement substitué par =O ou par un groupe alkyle ayant le sens indiqué ci-dessus pour $R^2$ et $R^3$, et/ou condensé avec un noyau benzène, furanne, thiophène ou pyrrole non substitué ou substitué comme indiqué ci-dessus pour les restes aryles, ou

$R^2$ a le sens indiqué ci-dessus et $R^1$ et $R^3$ forment ensemble un groupe alkylène ayant 2 à 5 atomes de carbone, éventuellment interrompu par 1 ou 2 -O-, -S- ou -$NR^4$, et/ou éventuellement substitué par =O ou par un groupe alkyle tel que défini dans le cas de $R^2$ et $R^3$, ou un groupe alkylène ayant 2 à 5 atomes de carbonne condensé avec un noyau benzène, furanne, thiophène ou pyrrole, non substitué ou substitué comme défini ci-dessus et * représente surtout une configuration R ou S, par hydrogénation catalytique asymétrique de cétimines prochirales de formule II

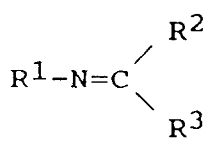

(II),

[dans laquelle $R^1$, $R^2$ et $R^3$ ont les sens indiqués ci-dessus], en présence de sels complexes d'un métal noble avec des ligands chiraux, procédé caractérisé en ce qu'on effectue l'hydrogénation à une température de -20 à + 80 °C et sous une pression d'hydrogène de $10^5$ Pa à $10^7$ Pa, et en ce qu'on ajoute au mélange réactionnel des quantités catalytiques d'un composé d'iridium de formule III ou IIIa

[XIrYZ] (III) ou $[XIrY]^{\oplus} + A^-$ (IIIa)

dans lesquelles X représente deux ligands oléfiniques ou un ligand du type diène, Y est une diphosphine chirale dont les groupes phosphines secondaires sont reliés par 2 à 4 atomes de carbone et qui forment avec l'atome de Ir un noyau pentagonal, hexagonal ou heptagonal, ou bien Y représente un diphosphinite chiral, dont les groupes phosphinites sont reliés par l'intermédiaire de 2 atomes de carbone et qui, avec l'atome de Ir, forment un noyau heptagonal; Z représente Cl, Br ou Pi, et $A^-$ représente l'anion d'un acide oxygéné ou complexe.

**2.** Procédé selon la revendication 1, caractérisé en ce que la température de réaction vaut de -20 à + 50 °C.

**3.** Procédé selon la revendication I, caractérisé en ce que la pression d'hydrogène vaut de $2.10^5$ Pa à $6.10^6$ Pa.

4. Procédé selon la revendication 1, caractérisé en ce que X représente, dans les formules III et IIIa, deux groupes éthylène ou un groupe diène en chaîne ouverte ou cyclique dont les doubles liaisons sont reliées par 1 ou 2 atomes de carbone.

5. Procédé selon la revendication 4, caractérisé en ce que le diène est l'hexadiène, le norbornadiène ou le cyclooctadiène.

6. Procédé selon la revendication 1, caractérisé en ce que, dans les formules III et IIIa, les symboles Y représentent une diphosphine chirale, dont les groupes phosphines sont reliés par 4 atomes de carbone et qui forment, avec l'atome de Ir, un noyau heptagonal.

7. Procédé selon la revendication 1 caractérisé en ce que les groupes phosphines et phoshinites contiennent un reste alkyle en $C_1$ à $C_{12}$, cycloalkyle ayant 5 à 8 atomes de carbone de noyau (qui peut être substitué par 1 à 3 groupes alkyles en $C_1$ à $C_6$), phényle, phénylalkyle en $C_7$ à $C_{12}$, ou alkylphénylalkyle ayant 1 à 6 atomes de carbone dans les groupes alkyles et 1 à 5 atomes de carbone dans les groupes alkylènes.

8. Procédé selon la revendication 1, caractérisé en ce que $A^{\ominus}$ représente un groupe $ClO_4^{\ominus}$, $CF_3SO_3^{\ominus}$, $BF_4^{\ominus}$, B(phényle)$_4^{\ominus}$, $PF_6^{\ominus}$, $SbCl_6^{\ominus}$, $AsF_6^{\ominus}$ ou $SbF_6^{\ominus}$.

9. Procédé selon la revendication 1 , caractérisé en ce que l'on ajoute le composé d'iridium en une quantité de 0,01 à 5 moles %, par rapport au composé de formule II.

10. Procédé selon la revendication 1, caractérisé en ce qu'on ajoute en outre un chlorure, bromure ou iodure d'ammonium ou de métal alcalin.

11. Procédé selon la revendication 1, caractérisé en ce que, dans la formule III, le symbole X représente un groupe cyclooctadiène, Z représente Cl et Y représente une diphosphine chirale.